# EUROPEAN PATENT APPLICATION

(11) **EP 4 488 689 A1**
(43) Date of publication of application: **08.01.2025**
(21) Application number: 23759597.0
(22) Date of filing: 31.01.2023
(51) Int. Cl.: G01N 35/00

(54) **AUTOMATED ANALYSIS DEVICE**

(30) Priority: 28.02.2022 JP 2022029233
(71) Applicant: HITACHI HIGH-TECH CORPORATION, Tokyo 105-6409 (JP); F. Hoffmann-La Roche AG, 4070 Basel (CH); Roche Diagnostics GmbH, 68305 Mannheim (DE)
(72) Inventor: TSUSHIMA Yuki, Tokyo 105-6409 (JP); USUI Takafumi, Tokyo 105-6409 (JP); ASCHENBRENNER Daniel, 68305 Mannheim (DE); BOEHM Julia, 68305 Mannheim (DE); HINZ Daniela, 68305 Mannheim (DE); STEIGENBERGER Maximilian, 68305 Mannheim (DE)
(74) Representative: Strehl Schübel-Hopf & Partner
(86) International application number: PCT/JP2023/002996
(87) International publication number: WO 2023/162591

(57) **Abstract**

To provide an automatic analyzer that prevents shutdown from being started while a reagent is left behind. The automatic analyzer includes: a reagent container holding unit configured to hold a reagent container housing a reagent; a reagent container information detection unit configured to detect information related to the reagent container; a display unit configured to display a screen including an operation region configured to receive an operation from a user; and a control unit configured to cause the display unit to display the screen. The control unit includes a container presence and absence determination unit configured to determine whether the reagent container is present in the reagent container holding unit based on the information detected by the reagent container information detection unit, a first operation region output unit configured to output to the screen a first operation region that is operated when the user confirms that the reagent container is taken out, a second operation region output unit configured to output to the screen a second operation region that is operated at the time of shutdown, and a warning output unit configured to output to the screen a warning, which prompts taking out of the reagent container, when the container presence and absence determination unit determines that the reagent container is present.

## Description

### Technical Field

The present invention relates to an automatic analyzer.

### Background Art

An automatic analyzer that analyzes a biological sample such as blood or urine performs the analysis by adding and mixing a reagent, which reacts to a target component, to and with the sample. In addition, in the automatic analyzer, when the planned analysis is completed, shutdown processing is performed by an operation of a user or the like in predetermined timing. Patent Document 1, for example, includes a description to the effect that the shutdown processing is stopped and a warning message is displayed when it is determined that a sample container is installed in the apparatus.

### Prior Art Document

### Patent Document

Patent Document 1: JP-2013-72744-A

### Summary of the Invention

### Problem to be Solved by the Invention

In the technology described in Patent Document 1, determination whether or not the sample container is present is made, but determination whether or not a reagent container is present is not made. The reagent needs to be used and stored in a state in which the temperature of the reagent is held constant in order to maintain an optimum state of the reagent. Hence, shutting down the automatic analyzer while the reagent container is left behind invites evaporation or degradation of the reagent. In addition, the shutdown processing is started temporarily irrespective of the presence or absence of the container. Therefore, when the container has been installed, the shutdown processing is stopped, and there is a possibility of taking time to restart the shutdown processing. There is a fear of burdening an unaccustomed user, in particular, when the stopping of the shutdown processing and the warning message occur frequently. Further, there is no opportunity to notice that the container is left behind until the shutdown processing is stopped and the warning message is issued. Thus, the container may be left as it is without the shutdown processing being completed when the user goes away from the apparatus immediately after the start of the shutdown processing.

It is an object of the present invention to provide an automatic analyzer that prevents a shutdown from being started while a reagent is left behind.

### Means for Solving the Problem

In order to solve the above-described problems, an automatic analyzer according to the present invention includes: a reagent container holding unit configured to hold a reagent container housing a reagent; a reagent container information detection unit configured to detect information related to the reagent container; a display unit configured to display a screen including an operation region configured to receive an operation from a user; and a control unit configured to cause the display unit to display the screen, in which the control unit includes a container presence and absence determination unit configured to determine whether the reagent container is present in the reagent container holding unit based on the information detected by the reagent container information detection unit, a first operation region output unit configured to output to the screen a first operation region that is operated when the user confirms that the reagent container is taken out, a second operation region output unit configured to output to the screen a second operation region that is operated at the time of shutdown, and a warning output unit configured to output to the screen a warning, which prompts taking out of the reagent container, when the container presence and absence determination unit determines that the reagent container is present.

### Advantages of the Invention

According to the present invention, it is possible to provide an automatic analyzer that prevents a shutdown from being started while a reagent is left behind.

### Brief Description of Drawings

[FIG. 1] FIG. 1 is a top view showing a schematic configuration of an automatic analyzer.
[FIG. 2] FIG. 2 is a functional block diagram showing an outline of a configuration of a control device.
[FIG. 3] FIG. 3 is a perspective view showing a configuration of a reagent container.
[FIG. 4] FIG. 4 is a flowchart showing processing until an automatic analyzer according to a first embodiment starts shutdown.
[FIG. 5A] FIG. 5A is an example of a GUI during transition to a shutdown screen.
[FIG. 5B] FIG. 5B is an example of the GUI when a take-out confirmation button is inactivated and a shutdown execution button is activated.
[FIG. 5C] FIG. 5C shows an example of the GUI when the take-out confirmation button and the shutdown execution button are inactivated.
[FIG. 5D] FIG. 5D shows an example of the GUI displaying a warning message prompting taking out of the reagent container.
[FIG. 6] FIG. 6 is a flowchart showing processing until an automatic analyzer according to a second embodiment starts shutdown.

### Modes for Carrying Out the Invention

An embodiment of the present invention will hereinafter be described with reference to the drawings.

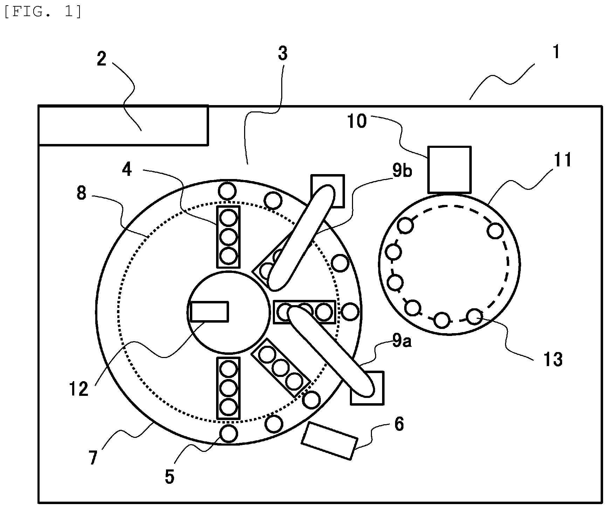

FIG. 1 is a top view illustrating a general configuration of an automatic analyzer 1. The automatic analyzer 1 includes a reagent sample disk 3 (reagent sample common holding unit), a reaction disk 11 (incubator), dispensing mechanisms (a first dispensing mechanism 9a and a second dispensing mechanism 9b), a spectrophotometer 10, an IC reader 12 (reagent container information detection unit), a barcode reader 6, and a control device 2.

The reagent sample disk 3 is mounted with reagent containers 4 containing reagents and sample containers 5 containing biological samples (samples) such as blood or urine, and is rotated by a driving mechanism not illustrated in the figure. In addition, the inside diameter side of the reagent sample disk 3 is provided with a reagent container holding unit 8 that holds the reagent containers 4. The outside diameter side of the reagent sample disk 3 is provided with a transport unit 7 that can transport a sample rack (not illustrated) in which the sample containers 5 are installed independently of the reagent container holding unit 8.

The reaction disk 11 is circumferentially mounted with a plurality of reaction containers 13, and is rotated by a driving mechanism not illustrated in the figure.

The first dispensing mechanism 9a dispenses a reagent and a sample for a biochemical test, for example. The second dispensing mechanism 9b dispenses a reagent and a sample for an immunological test, for example. Each of the dispensing mechanisms is provided between the reagent sample disk 3 and the reaction disk 11, and is capable of rotation and up and down movement. When dispensing a reagent, each dispensing mechanism aspirates the reagent from a reagent container 4 located at a reagent aspiration position on the reagent sample disk 3, and discharges the aspirated reagent into a reaction container 13 located at a dispensing position on the reaction disk 11. When dispensing a sample, each dispensing mechanism aspirates the sample from a sample container 5 located at a sample aspiration position on the reagent sample disk 3, and discharges the aspirated sample into the reaction container 13 located at the dispensing position on the reaction disk 11.

The spectrophotometer 10 is disposed on the periphery of the reaction disk 11. The spectrophotometer 10 performs measurement for a biochemical test. The spectrophotometer 10 includes a light source and a detector not illustrated in the figure. The spectrophotometer 10 measures the absorbance of a reaction solution in which the reagent and the sample are mixed with each other, by dispersing and detecting transmitted light obtained by irradiating the reaction solution with the light source. Incidentally, though not illustrated in the figure, the automatic analyzer 1 also includes, as a detecting mechanism for an immunological test, a detector that detects, by a photomultiplier tube, an amount of light originating from a luminescent reaction of a labeled substance included in the reaction solution made to react for a predetermined time by the reaction disk 11.

The IC reader 12 is provided to the inner circumferential side of the reagent container holding unit 8. The IC reader 12 reads an IC tag 14 as an identifier attached to the reagent container 4, and is also capable of determining the presence or absence of the reagent container 4 on the basis of whether or not the reading can be performed. Incidentally, in a case where the identifier attached to the reagent container 4 is an RFID tag or a two-dimensional barcode or the like other than the IC tag, a device capable of reading the identifier is used as the reagent container information detection unit. In addition, the reagent container information detection unit may detect information about the reagent container 4 other than the identifier as long as the reagent container information detection unit can detect whether or not the reagent container 4 is present in the reagent container holding unit 8. For example, the reagent container information detection unit may be a switch that is ON when the reagent container 4 is present, and is OFF when the reagent container 4 is not present, or the reagent container information detection unit may be a sensor that determines the presence or absence of the reagent container 4 on the basis of whether or not emitted light is interrupted.

The barcode reader 6 is disposed on the periphery of the reagent sample disk 3. The barcode reader 6 reads barcodes attached to the sample containers 5 and the sample rack.

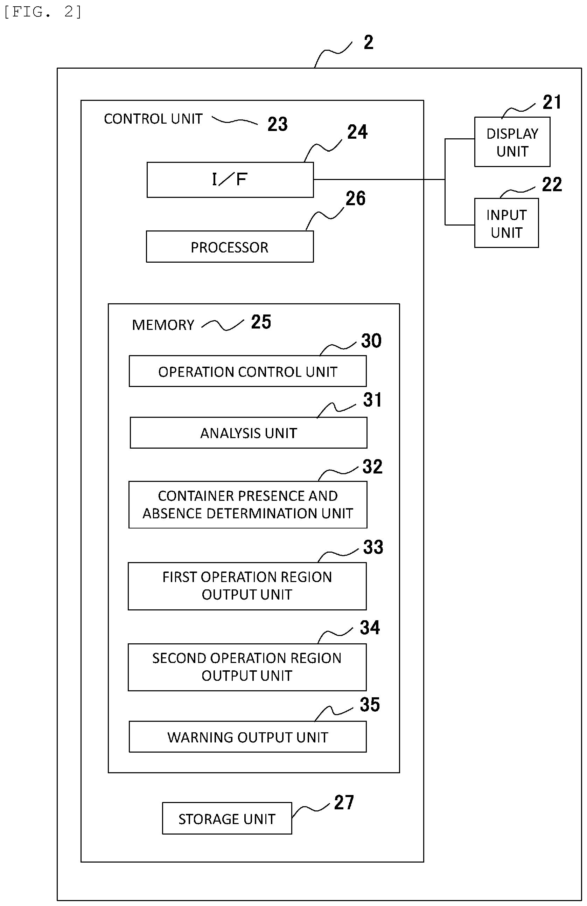

The control device 2 will next be described in detail. FIG. 2 is a functional block diagram illustrating an outline of a configuration of the control device. The control device 2 includes a display unit 21, an input unit 22, and a control unit 23.

The display unit 21 displays a screen including a state of the automatic analyzer 1, analysis items and analysis results, an operation region for receiving an operation from a user, a warning message at a time of an error, or the like. The display unit 21 is a display, for example. The input unit 22 is used when the user inputs a setting item or selects a predetermined display operation region. The input unit 22 is a keyboard and a mouse, for example. The display unit 21 and the input unit 22 may be formed integrally with each other as in a case of a touch panel. The control unit 23 is connected to the above-described mechanisms and the like constituting the automatic analyzer 1, controls the operation of the mechanisms and the like, and displays a screen on the display unit 21. Specifically, the control unit 23 includes an I/F 24, a memory 25, a processor 26, and a storage unit 27.

The I/F 24 is an interface that communicates with the display unit 21 and the input unit 22. The memory 25 stores various kinds of programs. The memory 25 is conceptually divided as, for example, an operation control unit 30, an analysis unit 31, a container presence and absence determination unit 32, a first operation region output unit 33, a second operation region output unit 34, and a warning output unit 35 for respective functions. The processor 26 executes the various kinds of programs stored in the memory 25. The processor 26 is constituted by a CPU or an MPU, for example. The storage unit 27 stores an operation sequence, an analysis result, and the like. The storage unit 27 is constituted by a storage device such as an HDD.

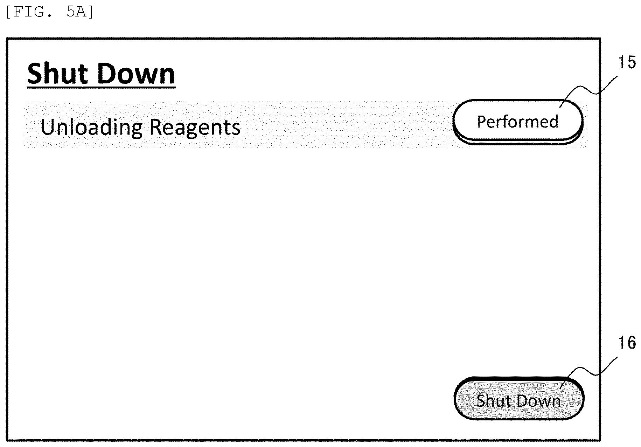

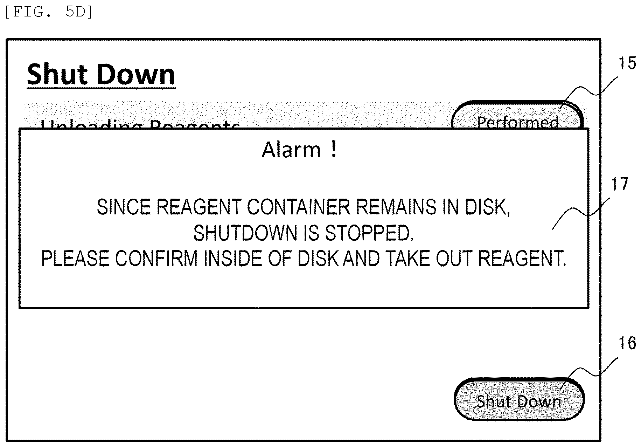

Here, the respective functions of the programs stored in the memory 25 will be described. The operation control unit 30 controls the operation of each mechanism, which is the rotational operation of the reagent sample disk 3 and the reaction disk 11, the dispensing operation of the dispensing mechanisms, and the like. The analysis unit 31, for example, calculates a component concentration of the sample on the basis of the absorbance measured by the spectrophotometer 10. The container presence and absence determination unit 32 determines whether or not the reagent container 4 is present in the reagent container holding unit 8 based on information detected by the reagent container information detection unit (IC reader 12). The first operation region output unit 33 outputs to the screen a first operation region (for example, a take-out confirmation button 15 in FIG. 5A or the like) that is operated when the user confirms that the reagent container 4 is taken out. The second operation region output unit 34 outputs to the screen a second operation region (for example, a shutdown execution button 16 in FIG. 5A or the like) that is operated at the time of shutdown. The warning output unit 35 outputs to the screen a warning (for example, a warning message 17 in FIG. 5D), which prompts taking out of the reagent container 4, when the container presence and absence determination unit 32 determines that the reagent container 4 is present.

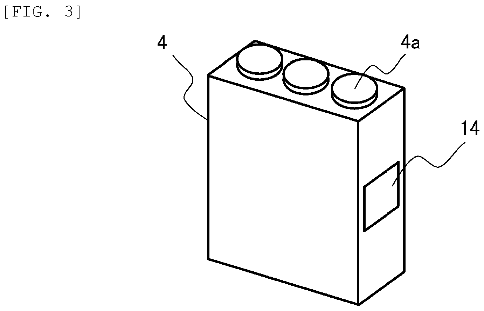

FIG. 3 is a perspective view illustrating a configuration of a reagent container. The reagent container 4 has a rectangular parallelepipedic shape. A plurality of opening portions 4a for each dispensing mechanism to access the reagent are provided to the upper surface of the reagent container 4 so as to protrude upward. An IC tag 14 is attached to one of short-side side surfaces of the reagent container 4. Incidentally, the reagent container 4 is mounted in the reagent container holding unit 8 such that the side surface provided with the IC tag 14 faces the IC reader 12 on the inside diameter side. Reagent information related to the reagent contained in the reagent container 4 is recorded in the IC tag 14. The reagent information includes a kind of the reagent, a lot number, an individual identification number, a reagent remaining amount, a test quantity, a date and time of a start of usage of the reagent container, a time limit on the usage of the reagent, and the like. Incidentally, as described earlier, the identifier attached to the reagent container 4 may be an RFID tag, a two-dimensional barcode, or the like.

### <First Embodiment>

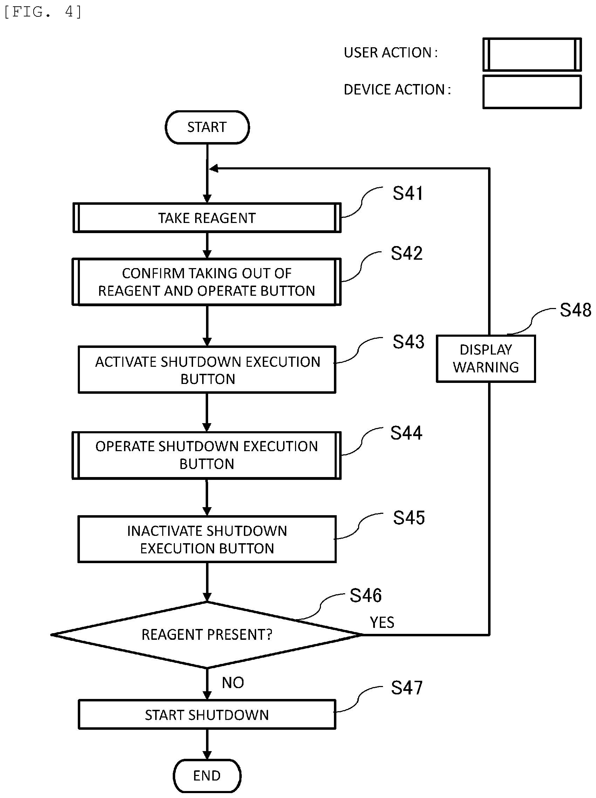

FIG. 4 is a flowchart illustrating processing up to a start of a shutdown by the automatic analyzer according to a first embodiment. In FIG. 4, steps S41, S42, and S44 are processing by the user, and steps S43 and S45 to S48 are processing by the automatic analyzer 1 (control unit 23).

When analysis is completed, and the user performs a predetermined operation such as tapping a shutdown selection button (not illustrated) in a home menu, the control unit 23 effects a transition of the screen output on the display unit 21 to a shutdown screen. FIG. 5A illustrates an example of a GUI during transition to the shutdown screen. In the GUI illustrated in FIG. 5A, the take-out confirmation button 15 is set so as to receive an operation by the user (is activated). On the other hand, the shutdown execution button 16 is set so as not to receive an operation by the user (is inactivated). Incidentally, the shutdown execution button 16 may be set so as not to be displayed instead of being inactivated.

Next, according to the screen of FIG. 5A, the user takes out the reagent container 4 from the reagent container holding unit 8 when the reagent container 4 remains in the reagent container holding unit 8 (step S41). When the user completes the takeout, the user operates the take-out confirmation button 15 in FIG. 5A (step S42).

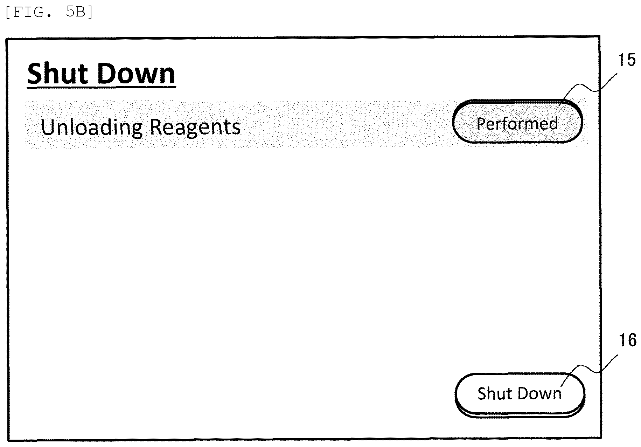

When the take-out confirmation button 15 is operated, the first operation region output unit 33 inactivates the take-out confirmation button 15, and the second operation region output unit 34 activates the shutdown execution button 16 (step S43). FIG. 5B illustrates an example of the GUI when the take-out confirmation button is inactivated and the shutdown execution button is activated.

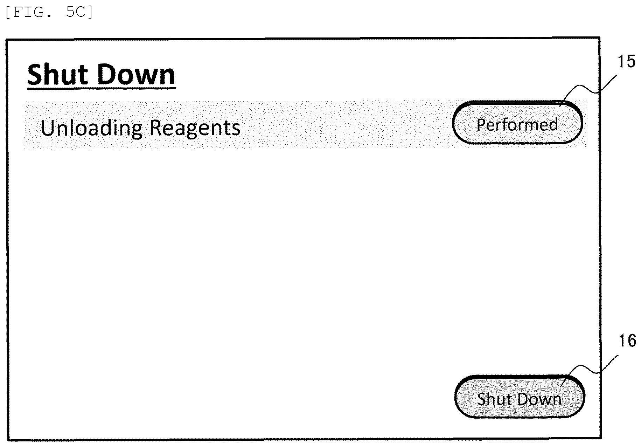

When the shutdown execution button 16 is activated, the user operates the shutdown execution button 16 (step S44). When the shutdown execution button 16 is operated, the shutdown execution button 16 is inactivated again (step S45). The container presence and absence determination unit 32 determines whether or not the reagent container 4 is present based on whether or not the IC reader 12 has succeeded in reading the IC tag 14 (step S46). FIG. 5C illustrates an example of the GUI when the take-out confirmation button and the shutdown execution button are inactivated. When the IC reader 12 cannot read the IC tag 14, and thus the container presence and absence determination unit 32 determines that the reagent container 4 is absent, the control unit 23 starts to shut down the automatic analyzer 1 (step S47).

When, in step S46, the IC reader 12 has succeeded in reading the IC tag 14, and thus the container presence and absence determination unit 32 determines that the reagent container 4 is present, the warning output unit 35 outputs to the screen the warning message 17, which prompts taking out of the reagent container 4 (step S48). FIG. 5D illustrates an example of the GUI displaying the warning message, which prompts taking out of the reagent container.

The present embodiment can prevent the automatic analyzer 1 from being shut down while the reagent container 4 containing the reagent remains, and can therefore suppress evaporation or degradation of the reagent. In addition, the shutdown execution button 16 is inactivated unless the take-out confirmation button 15 is operated. The user can therefore be given an opportunity to notice that the reagent container 4 is left behind before a start of shutdown processing. As a result, even for an unaccustomed user, it is possible to suppress frequent occurrence of a situation in which the shutdown processing is stopped halfway and the warning message 17 is displayed.

### <Second Embodiment>

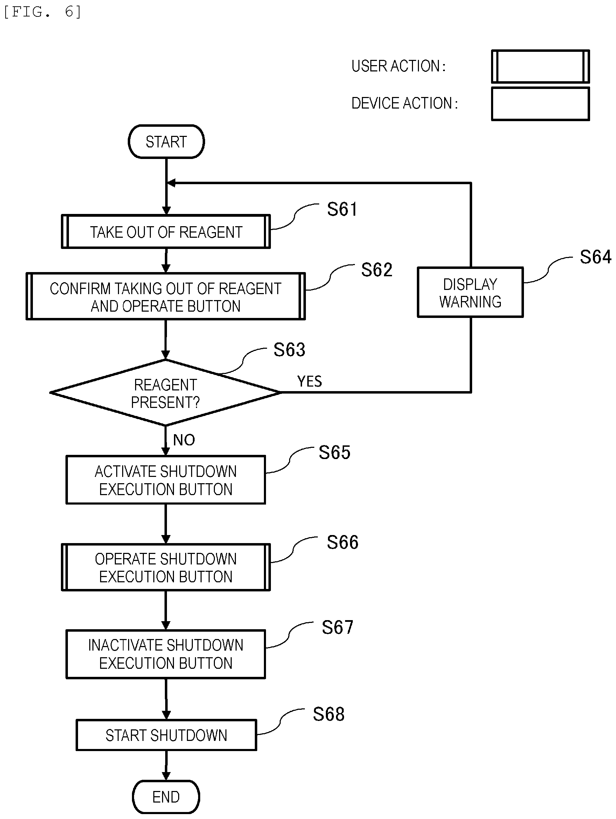

FIG. 6 is a flowchart illustrating processing up to a start of shutdown performed by the automatic analyzer according to a second embodiment. In FIG. 6, steps S61, S62, and S46 are processing by the user, and steps S63 to S65, S67, and S68 are processing by the automatic analyzer 1 (control unit 23).

Also in the present embodiment, when analysis is completed, and the user performs a predetermined operation, the control unit 23 outputs the shutdown screen illustrated in FIG. 5A to the display unit. Then, according to the screen of FIG. 5A, when the reagent container 4 remains in the reagent container holding unit 8, the user takes out the reagent container 4 from the reagent container holding unit 8 (step S61). When the user completes the takeout, the user operates the take-out confirmation button 15 in FIG. 5A (step S62). The second embodiment is thus far similar to the first embodiment.

Next, when the take-out confirmation button 15 is operated, the first operation region output unit 33 inactivates the take-out confirmation button 15, and the container presence and absence determination unit 32 determines whether or not the reagent container 4 is present (step S63). When the container presence and absence determination unit 32 determines that the reagent container 4 is present, the warning output unit 35 outputs to the screen the warning message 17 as illustrated in FIG. 5D, which prompts taking out of the reagent container 4 (step S64) .

When the container presence and absence determination unit 32 determines in step S63 that the reagent container 4 is absent, the second operation region output unit 34 activates the shutdown execution button 16, as illustrated in FIG. 5B (step S65). Then, the user operates the shutdown execution button 16 (step S66). When the shutdown execution button 16 is operated, the shutdown execution button 16 is inactivated again, as illustrated in FIG. 5C (step S67), and the control unit 23 starts to shut down the automatic analyzer 1 (step S68).

As with the first embodiment, the present embodiment can prevent the automatic analyzer 1 from being shut down while the reagent container 4 containing the reagent remains, and can therefore suppress evaporation or degradation of the reagent. In addition, in the present embodiment, the shutdown execution button 16 is activated for the first time after the container presence and absence determination unit 32 confirms that the reagent container 4 does not remain. It is therefore possible to prevent the occurrence of a situation in which the shutdown is stopped after the user who has operated the shutdown execution button 16 goes away from the apparatus, and the state is left as it is.

Embodiments have been described above. However, the present invention is not limited to the foregoing embodiments, but includes various modifications. For example, the automatic analyzer according to each embodiment displays the take-out confirmation button 15 and the shutdown execution button 16 as different buttons, but may display the take-out confirmation button 15 and the shutdown execution button 16 as a common button as in a case where the take-out confirmation button 15 changes to the shutdown execution button 16 when the take-out confirmation button 15 is operated, for example.

### Reference Sign List

- 1:: automatic analyzer
- 2:: control device
- 3:: reagent sample disk
- 4:: reagent container
- 4a:: opening
- 5:: sample container
- 6:: barcode reader
- 7:: transport unit
- 8:: reagent container holding unit
- 9a:: first dispensing mechanism
- 9b:: second dispensing mechanism
- 10:: spectrophotometer
- 11:: reaction disk
- 12:: IC reader
- 13:: reaction container
- 14:: IC tag
- 15:: take-out confirmation button
- 16:: shutdown execution button
- 17:: warning message
- 21:: display unit
- 22:: input unit
- 23:: control unit
- 24:: I/F
- 25:: memory
- 26:: processor
- 27:: storage unit
- 30:: operation control unit
- 31:: analysis unit
- 32:: container presence and absence determination unit
- 33:: first operation region output unit
- 34:: second operation region output unit
- 35:: warning output unit

## Claims

1. An automatic analyzer comprising:
a reagent container holding unit configured to hold a reagent container housing a reagent;
a reagent container information detection unit configured to detect information related to the reagent container;
a display unit configured to display a screen including an operation region configured to receive an operation from a user; and
a control unit configured to cause the display unit to display the screen, wherein
the control unit includes
a container presence and absence determination unit configured to determine whether the reagent container is present in the reagent container holding unit based on the information detected by the reagent container information detection unit,
a first operation region output unit configured to output to the screen a first operation region that is operated when the user confirms that the reagent container is taken out,
a second operation region output unit configured to output to the screen a second operation region that is operated at the time of shutdown, and
a warning output unit configured to output to the screen a warning, which prompts taking out of the reagent container, when the container presence and absence determination unit determines that the reagent container is present.

2. The automatic analyzer according to claim 1, wherein
when the first operation region is operated, the second operation region is activated.

3. The automatic analyzer according to claim 2, wherein
when the activated second operation region is operated, determination of the container presence and absence determination unit is performed.

4. The automatic analyzer according to claim 3, wherein
the shutdown is started when the container presence and absence determination unit determines that the reagent container is absent.

5. The automatic analyzer according to claim 1, wherein
when the first operation region is operated, the determination of the container presence and absence determination unit is performed while the second operation region is inactivated.

6. The automatic analyzer according to claim 5, wherein
the second operation region is activated when the container presence and absence determination unit determines that the reagent container is absent.

7. The automatic analyzer according to claim 6, wherein
the shutdown is started when the activated second operation region is operated.

8. The automatic analyzer according to claim 1, wherein
the reagent container information detection unit is a reading device that reads an identifier attached to the reagent container.

9. An automatic analyze, comprising:
a reagent container holding unit configured to hold a reagent container housing a reagent;
a display unit configured to display a screen including an operation region configured to receive an operation from a user; and
a control unit configured to cause the display unit to display the screen, wherein
the control unit includes
a first operation region output unit configured to output to the screen a first operation region that is operated when the user confirms that the reagent container is taken out, and
a second operation region output unit configured to output to the screen a second operation region that is operated at the time of shutdown, and
the second operation region is activated after the first operation region is operated.
